Europäisches Patentamt

European Patent Office

Office européen des brevets

(11) Publication number: **0 535 237 A1**

# EUROPEAN PATENT APPLICATION
## published in accordance with Art. 158(3) EPC

(21) Application number: 92906210.7

(22) Date of filing: 28.02.92

(86) International application number:
PCT/JP92/00225

(87) International publication number:
WO 92/16237 (01.10.92 92/25)

(51) Int. Cl.⁵: **A61K 47/14**, A61K 7/00

(30) Priority: 20.03.91 JP 57367/91
20.03.91 JP 57368/91
20.03.91 JP 57369/91
10.05.91 JP 106129/91

(43) Date of publication of application:
07.04.93 Bulletin 93/14

(84) Designated Contracting States:
AT BE CH DE DK FR GB IT LI NL SE

(71) Applicant: HISAMITSU PHARMACEUTICAL CO. INC.
408, Tashirodaikanmachi
Tosu-shi Saga 841(JP)

(72) Inventor: SATO, Syuji Tsukuba Institute of
Hisamitsu Pharm. Co. Inc 25-11, Kannondai
1-chome
Tsukuba-shi Ibabaragi 305(JP)
Inventor: MUKAE, Katsuya Tsukuba Institute
of
Hisamitsu Pharm. Co. Inc 25-11, Kannondai
1-chome
Tsukuba-shi Ibaragi 305(JP)
Inventor: HIGASHI, Yoshinobu Tsukuba

Institute of
Hisamitsu Pharm. Co. Inc 25-11, Kannondai
1-chome
Tsukuba-shi Ibaragi 305(JP)
Inventor: SUZUKI, Tatsuaki Tsukuba Institute
of
Hisamitsu Pharm. Co. Inc 25-11, Kannondai
1-chome
Tsukuba-shi Ibaragi 305(JP)
Inventor: MIZUMACHI, Mitsuhiro Tsukuba
Institute of
Hisamitsu Pharm. Co. Inc 25-11, Kannondai
1-chome
Tsukuba-shi Ibaragi 305(JP)
Inventor: SAKAGUCHI, Hirofumi Tsukuba
Institute of
Hisamitsu Pharm. Co. Inc 25-11, Kannondai
1-chome
Tsukuba-shi Ibaragi 305(JP)

(74) Representative: Holmes, Michael John
Frank B. Dehn & Co. European Patent
Attorneys Imperial House 15-19 Kingsway
London, WC2B 6UZ, (GB)

(54) COMPOSITION FOR RELIEVING SKIN IRRITATION AND EXTERNAL PREPARATION FOR PERCUTANEOUS ADMINSTRATION CONTAINING THE SAME.

(57) A composition for relieving skin irritation comprising 1 to 50 wt % of trialkyl citrate, 1 to 70 wt % of a lower alcohol and 1 to 90 wt % of water, and an external preparation for percutaneous administration comprising the above composition and further containing 0.001 to 20 wt % of a pharmacologically active substance, both of which can lower and relieve skin irritation and promote the absorption of the pharmacologically active substance.

FIELD OF THE INVENTION

The present invention relates to a skin irritation mitigating composition and a percutaneous external preparation. More particularly, the present invention relates to a novel composition and a percutaneous external preparation having the function of reducing or mitigating skin irritation as well as the function of accelerating absorption of pharmaceutically active components.

PRIOR ART

Drugs have conventionally been administered orally or by injection; these routes have the problems of side effects such as gastric disturbance and shock. To alleviate these side effects caused by drug medicines, research has recently been carried out on external preparations which permit percutaneous administration of medication. Up till now, however, administration of external preparations has not given a sufficient percutaneous absorption in many cases.

In order for a drug to display its maximum therapeutic effect through percutaneous administration, the drug must quickly move from the basal medium into the interior or skin and thus reach the affected part. To achieve this objective, various contrivances have so far been made to improve percutaneous absorption of a medication dispersed or dissolved in the basal medium. It is proposed, for example, to combine two or more kinds of solvent, as disclosed in Japanese Patent Provisional Publication No. 61-249,934, and to add a percutaneous absorption accelerator such as an azacycloalkane derivative, as disclosed in Japanese Patent Provisional Publication No. 62-238,261. Many of these proposals are however defective in that the general application or usage is limited by the nature as a strong solvent or by a serious skin irritation caused by the percutaneous absorption accelerator itself. Various trials have therefore been made with a view to solving these problems: active research efforts have been made on the addition of a particular chemical substance for alleviating skin irritation, as disclosed in Japanese Patent Provisional Publication No. 58-4,721, Japanese Patent Provisional Publication No. 60-56,911, and Japanese Patent Provisional Publication No. 60-23,312, and more recent proposals include a method of combining a non-steroidal antiinflammatory agent as disclosed in Japanese Patent Provisional Publication No. 1-299,212, a method of combining an azene derivative as disclosed in Japanese Patent Provisional Publication No. 1-299,217, a technique based on a composition containing glycerine as disclosed in Japanese Patent Provisional Publication No. 63-211,241, and a method of adding 2-phenyl-1-ethanol, and 3-phenyl-1-propanol as a metabolic adjuster as disclosed in Japanese Patent Provisional Publication No. 2-96,514. Problems remain however as to the practicability of these proposed methods.

On the other hand, trialkyl citrate, or triethyl citrate in particular, is popularly employed as a basal medium for cosmetics as disclosed in Japanese Patent Publication No. 3-25,403, Japanese Patent Provisional Publication No. 64-19,012 and Japanese Patent Provisional Publication No. 64-66,107 because of its excellent feeling of use and low skin toxicity. It is furthermore known to be capable of reducing skin irritation of a perfume by reducing the penetration of the perfume into skin (J. Soc. Cosmet. Chem. Japan, Vol. 19, No. 1, p. 30, 1985).

However, its application in medical drugs is limited: its utilization is limited to addition as a plasticizer or an emollient as disclosed in Japanese Patent Provisional Publication No. 1-113,055, Japanese Patent Provisional Publication No. 1-113,056, Japanese Patent Provisional Publication No. 2-184,622, and Japanese Patent Provisional Publication No. 2-237,917, and to usage as a stabilizing solvent for prostaglandin as disclosed in the specification of the United States Patent No. 4,515,810. Its use as a percutaneous absorption accelerating administrative composition is disclosed only in the specification of the United States Pantent No. 4,666,926, Japanese Patent Provisional Publication No. 63-126,832 and in the specification of the United States Patent No. 4,814,173. Medications are however limited in the specification of the United States Pantent No. 4,666,926, and no description is given as to its skin irritation in any of these cases.

Various contrivances and proposals have been made regarding external preparations as described above, and percutaneous external drugs are now among the most popularly used preparations, and although their serious side effects are not yet completely known, adverse effects such as skin irritation are only increasing, and no steps are being taken to deal with this at present.

The present invention was made in view of the circumstances as described above, and has an object to provide a novel composition and externally applied drugs using the compositions which solve the problems associated with the conventional externally applied drugs and prescription thereof and permits, through prescription of a specific basal medium, reduction and mitigation of irritation and achievement of the function of accelerating absorption.

## DISCLOSURE OF THE INVENTION

As a means to solve the above-mentioned problems, the present invention provides a composition useful for skin external drugs, which permits achievement of a very high percutaneous absorption and a very low skin irritation of the pharmaceutically active components by combining trialkyl citrate, water and low-grade alcohol, or combining an absorption accelerator into these basal media.

More specifically, the inventors of the present invention carried out extensive studies on various compositions with a view to solving the above-mentioned problems and found out the possibility of overcoming the drawbacks as described above by combining trialkyl citrate, water and a low-carbon alcohol. In addition, they found out the possibility of accelerating percutaneous absorption and alleviate skin irritation by combining an absorption accelerator in these basal media, thus completing the present invention.

Now, the composition of the present invention will be described in detail.

First, trialkyl citrate used in the present invention may be any of butyl, isopropyl, propyl, ethyl and methyl, among which trialkyl citrate taking the form of liquid at the ambient temperature and giving a good feeling of use is particularly preferable. The amount of trialkyl citrate to be combined is about 1 to 50 wt.% or more preferably, 5 to 30 wt.% relative to the entire composition as 100 wt.%. The preferable ratio of combination varies with the kind of low-carbon alcohol to be combined.

The low-carbon alcohol used in the present invention may be selected from among those having from 1 to 4 carbon atoms, or more preferably, from 2 to 3. More specifically, methyl alcohol, ethyl alcohol, n-propyl alcohol, isopropyl alcohol and n-butyl alcohol may be cited as preferable ones. The amount of combined low-carbon alcohol should be 1 to 10 wt.%, or more preferably, 20 to 50 wt.%, relative to the entire composition as 100 wt.%. The preferable ratio of combination varies with the kind of alcohol. Water used in the present invention may be any of purified water, salt water and buffer used for pH adjustment. Water selected from among these descriptions must be combined in an amount of 1 to 90 wt.%, or more preferably, 30 to 70 wt.% relative to the entire composition as 100 wt.%. The preferable ratio of combination varies with the kind of low-carbon alcohol to be combined.

Conceivable absorption accelerators include terpene compounds, fatty acids, fatty acid alcohol, fatty acid ester, phenol derivatives and azacycloalkane derivatives as mentioned below. Among terpene compounds, monoterpene alcohol is usually preferable. The terpene compound may be saturated or non-saturated and may be any of straight chain, branched and cyclic ones. When cyclic one is selected, monocyclic or bicyclic one is preferable. Typically preferable monoterpene compounds include, for example, geraniol, eugenol, isoeugenol, thymol, terpineol, L-menthol, borneol, isoborneol, nerol, citronellol, linallol, rhodinol, safrol, isosafrol, D-(+)-limonene, pinene, dl-comphor, camphene, menthone, and thymene.

Preferable fatty acid alcohol, fatty acid ester and phenol derivatives include, for example, caprylic acid, capric acid, caproic acid, oleic acid, linolic acid, myristic acid, salicylic acid, oleil oleate, palmitic acid, diethyl cinnamate, diethyl sebacate, sorbitan sesquioleate, methyl laurate, polyethylene glycol monostearate, oleil alcohol, myristic alcohol, butylhydroxy anisole, and chlorcresol.

Finally, examples of azacycloalkane derivatives include, for example, 1-[2-(decylthio) ethyl] azacyclopentane-2-on or 1-n-dodecylazacycloheptane-2-on, and the compounds as disclosed in Japanese Patent Provisional Publication No. 3-500,287 and Japanese Patent Provisional Publication No. 3-500,543. The absorption accelerator as described above may be combined in an amount of about 0.1 to 10%, or more preferably, 1 to 5% relative to the entire composition as 100 wt.%.

For the pharmaceutically active substances to be contained in the external composition of the present invention, furthermore, there is no particular limitation on the kind if it is a medication to be percutaneously absorbed, and applicable substances include, for example, general anesthetic agents, hypnotic/sedative agents, anti- anents, antipyretic.analgesic/antiinflammatory agents, analeptic agents, awaking agents, psycho-neuro agents, local anesthetic agents, skeletal muscle relaxing agents, autonomic agents, spasmolytic agents, anti-perkinsonism agents, ophthalmologic agents, otorhinologic agents, antihistamic agents, cardiotonicum, antihypertensive agents, vasoconstrictor, coronary vasodilator, peripheral vasodilator, circulatory organ agents, respiratory stimulant agents, antituesive and expecterant agents, digestive ulcer therapeutic agents, cholagogues, intestinal agents, thyrotropen/antithyroid hormone agents, narcotic, anabolic steriodal agents, adrenocorticotropic hormone agents, androgen agents, follicle luteinizing hormone agents, urologic and genital organ agents, anus agents, analgesic agents, antipruritic agents, astringents, antiinflammatory agents, parastic skin disease agents, skin emollients, corrosion agents, vitamin agents, blood coagulation inhibitors, habitual intoxication agents, antigout agents, enzyme preparations and diabetes mellitus agents, and among others, preferable ones include hypnotic and sedative agents, antipyretic/analgesic/antiflammatory agents, psycho-neuro agents, local anesthetic agents, skeletal muscle relaxing agents, autonomic agents, spasmeolytic agents, anti-Perkinsonism agents, antihistamine agents, cardiotonicum, antiphyertensive agents, vasoconstrictors, coronary vasodilators, peripheral vasodilators, cholagogues, antithyroidal hormone agents, narcotics, adrenocorticotropic hormone agents, follicle luteinizing hormone agents, parasitic skin disease agents, antigout agents, and diabetes mellitus agents. These medications should preferably be water-soluble, and it is desirable to adjust pH of the composition to 7 to 8 for an acidic medication, and to 4 to 7 for a basic medication. These medications may be used singly or in mixture of two or more thereof. An ultraviolet ray absorbing agent, anti-oxidation agent or antiseptic agent may be added as required.

Applicable ultraviolet absorbing substances include, for example, conventionally known p-amino benzoic acid derivatives, anthranilic acid derivatives, coumarin derivatives, aminoacid compounds, benzotriazole derivatives, tetrazole derivatives, imidozoline derivatives, pyrimidine derivatives, dioxane derivatives, furan derivatives, pyrone derivatives, camphor derivatives, nucleic acid derivatives, allantoin derivatives, nicotine derivatives, and siconine or vitamin $B_6$ derivatives, and a particularly preferable ones are benzophenone derivatives such as 2-hydroxy-4-methoxybenzophenone derivatives. Antioxidation agents include, for example, ascorbic acid, stearic acid ester, sodium ascorbate, tocopherol (d, 1 and d1 forms of $\alpha$-tocopherol, $\beta$-tocopherol, $\gamma$-tocopherol and $\delta$-tocopherol) and ester derivatives thereof, nordihydroguaceletic acid, dibutylhydroxy toluene, butylhydroxy anisole, tert-butyldyroquinone gallic acid ester (esters of ethyl, propyl and isoamyl), 1-oxo-3-methyl-4-isopropylbenzene, and other antioxidation agents. As the antiseptics, benzoic acid, sodium benzoate, paraoxy benzoate ester, paraoxy benzoate propyl, and paraoxy benzoate butyl are used.

These ultraviolet absorbing agents may be combined in an amount of about 0.01 to 5 wt.%, or more preferably, o.1 to 1 wt.% relative to the entire composition as 100 wt.%. The antioxidation agents may also be combined in an amount of about 0.01 to 5 wt.%, or more preferably, 0.1 to 1 wt.% relative to the entire composition as 100 wt.%. The antiseptics may be combined in an amount of about 0.01 to 5 wt.%, or more preferably, 0.05 to 1 wt.% relative to the entire composition as 100 wt.%.

As described above, the present invention permits containing percutaneously absorbable pharmaceutically active substances as active components, combination of trialkyl citrate, water and low-carbon alcohol with these active components, and hence simultaneous achievement of a high skin penetration or pharmaceutically effective substances and very low skin irritation. An embodiment is to combine an absorption accelerator with the above-mentioned basal media, and this permits simultaneous achievement of a high skin penetration of effective substances and a very low skin irritation. An excipient, an ultraviolet ray absorbant, and anti-oxidation agent, and an antiseptic may be combined as required. Conceivable forms of drug include gel, gel-cream, liniment, aerosol, reservoir type patch drug, and patch drugs.

Now, for each of the above-mentioned external drugs, examples of prescribed of the present invention will be presented below.

First, the gel-form basal medicine is described. In a gel-form one, basal media selected from the group consisting of trimethyl citrate, triethyl citrate, triisopropyl citrate, or tributyl citrate, and low-carbon alcohol (such as ethanol, and isopropanol), water, gellating agents (such as carboxyvinyl polymer, hydroxyethyl cellulose, hydroxypropyl cellulose, methyl cellulose and carboxy methylcellulose), neutralizing agents (such as triethanol amine, diisopropanol amine, and sodium hydrochloride), surfactants (such as sorbitan sesquioleate, sorbitan trioleate, sorbitan monooleate, sorbitan monostearate, sorbitan monolaurate, polyethylene glycol monostearate, polyoxyethylene cetyl ether, and polyoxyethylene lauril ether), and absorption accelerators (such as azacycloalkane derivatives) are appropriately combined, and anti-oxidation agents, ultraviolet absorbants and antiseptics are combined as required. The gel-form drug of the present invention is available by further combining pharmaceutically active substances.

A typical example of manufacture of a gel-form drug is now presented for reference.

| Pharmaceutically active substance | Appropiate quantity |
|---|---|
| Trialkyl citrate | up to 30 wt.%, |
| Water | up to 55 wt.%, |
| Glycol | up to 40 wt.%, |
| Low-carbon alcohol | up to 45 wt.%, |
| Gellating agent | up to 5 wt.%, |
| Absorption accelerator | up to 5 wt.%. |

These components are weighed and swelled by adding water. One the other hand, the pharmaceutically active substances are dissolved or suspended in a solvent in an appropriate amount, and the resultant

4

solution or suspension is dissolved in a mixture of a glycol and trialkyl citrate, a mixture of trialkyl citrate and a low-carbon alcohol, or a mixture of these three substances. Then, the resultant mixture is added to water swelled with a gellating agent, together with a neutralizing agent so as to give a pH of 4 to 8, thus leading to gel-form drug of the present invention. This embodiment of manufacture is only an example, and it is needless to mention that the drug of the present invention is also manufacturable by conventional or similar process and prescription. No particular limitation is set on the ratios of combination of the individual components.

Now, the gel-cream drug of the present invention is described below. Any of trimethyl citrate, triethyl citrate, triisopropyl citrate and tributyl citrate which are features of the present invention is appropriately combined with basal media selected from the group consisting of known cream basal media such as white vaseline, liquid paraffin, high-grade fatty acid esters (such as myristic acid ester, palmitic acid ester, sabacic acid ester, lauric acid hexyl, isooctic acid cepyl), low-carbon alcohol (such as ethanol and isopropanol), emulsifiers (such as polyoxyethylenealkyl ethers, fatty acid esters and polyethylene glycol fatty acid esters), absorption accelerators (such as terpene compounds and azacycloalkane derivatives), gelleting agents (such as carboxyvinyl polymers, hydroxyethyl cellulose, hydroxypropyl cellulose, methyl cellulose and carboxymethyl cellulose) and neutralizing agents (such as triethanol amine, diisopropanol amine and sodium hydrochloride), and an anti-oxidation agent, an ultraviolet ray absorbant, or an antiseptic is added as required. The gel-cream drug of the present invention is available by combining pharmaceutically active substances.

Now, an example of manufacture of a gel-cream drug is described below for reference.

| Pharmaceutically active substances | Appropriate quantity, |
|---|---|
| Trialkyl citrate | up to 20 wt%, |
| High-grade fatty acid ester | up to 25 wt.%, |
| Antiseptic | up to 0.5 wt.%, |
| Emulsifier | up to 5 wt.% |
| High-carbon alcohol | up to 15 wt.%, |
| Water | up to 50 wt.%, |
| Low-carbon alcohol | up to 20 wt.%, |
| Absorption accelerator | up to 5 wt.%, |
| Gellating agent | up to 5 wt.%. |

The above-listed components are weighed, and high-grade fatty acid ester, trialkyl citrate, high-carbon alcohol, emulsifier and absorption accelerator are first mixed, heated to 50 to 100°C for melting, and the thus heated mixture is added with antiseptic, low-carbon alcohol and pharmaceutically active substances dissolved or suspended in previously heated water. The resultant solution or suspension is then cooled to 50°C, and the gellating agent is added and caused to disperse sufficiently. Subsequently, a gel-cream of the present invention is available by adjusting pH to 4 to 8 through addition of a neutralizing agent. This embodiment of manufacture is only an example, and it is needless to mention that the drug of the present invention is also manufacturable by conventional or similar process and prescription. The ratios of the individual components are not limited to these presented above.

Now, the liniment agent is described below. An absorption accelerator (such as a terpene compound or an azacycloalkane derivative) and pharmaceutically active substances are added to 30 wt.% any of trimethyl citrate, triethyl citrate, triisopropyl citrate and tributyl citrate which are features of the present invention, 10 to 70 wt.% low-carbon alcohol (such ethanol or isopropanol) which is a conventional liniment basal medium up to 55 wt.% water, and up 60 wt.% fatty acid ester (such as an ester of adipic acid, sebacic acid or myristic acid), and 0.01 to 5 wt.% ultraviolet ray absorbant, 0.01 to 5 wt.% antioxidation agent and/or 0.01 to 5 wt.% antiseptic are combined as required, thus giving a liniment drug of the present invention. The example of prescription and the example of manufacture described above are only examples, and the liniment drug of the present invention is of course available with a similar prescription. Even in the liniment drug of the present invention, a neutralizing agent for adjusting pH or a viscosity imparting agent such as methyl cellulose, carboxyvinyl polymer or hydroxypropyl cellulose may be added.

Now, the aerosol drug is described below. The aerosol drug of the present invention is available by filling an aerosol contained under pressure with any of trimethyl citrate, triethyl citrate, triisopropyl citrate and tributyl citrate which are features of the present invention in an amount of up to 20 wt.%, low-carbon alcohol (such as ethanol or isopropanol) and purified water in an amount of up to 30 wt.%, an absorption accelerator (such as a terpene compound or an azacycloalkane derivative), pharmaceutically active substances, and as required an inorganic substance such as talc in an amount of up to 5%, an ultraviolet ray absorbant in an amount of 0.01 to 5 wt.%, and anti-oxidation agent in an amount of 0.01 to 5 wt.%, an antiseptic in an amount of 0.01 to 5 wt.%, dimethyl ether in an amount of up to 20 wt.% and LPG in an amount of up to 20 wt.%. The example of prescription and the example of manufacture as presented above are only examples, and the aerosol drug of the present invention is available of course with a similar prescription.

Now, the reservoir type patch drug is described below. Any of the compositions of the gel-form drug, the gel-cream drug and the liniment drug as described above and pharmaceutically active substances may be used as the composition for the reservoir fraction in this reservoir type patch drug. The control membrane in the form of a porous membrane or a macromolecular membrane may be made of any of polyethylene, polypropylene, polymethacrylic acid ester, polyurethane, polyester, polyvinyl alcohol-ethylene copolymer, polyvinyl alcohol, polyvinyl chloride, polyamide, ethylene-vinyl acetate copolymer, ehtylenic copolymer gum, ethylene-methacrylic acid and ethylene-acrylic acid copolymer. The support may be made of polypropylene polyester, polyvinylidene chloride, polyacryl, polyurethane, ethylene-vinyl acetate copolymer, cloth and non-woven cloth. The surface in contact with skin via the support reservoir portion, the porous membrane or the macromolecular membrane may be with or without an adherent layer. When an adherent layer is provided, it may be made of a patch drug composition containing pharmaceutically active substances. When an adherent layer is non-existent, the drug of the present ivnention is available by providing an adherent layer based on the basal media of the patch drug not containing pharmaceutically active substances outside the porous membrane of the macromolecular membrane, which is the releasing surface. The conventional method may be used for the manufacture of these components as described above.

Finally, the patch drug is described below. An absorption accelerator (such as an azacycloalkane derivative) and pharmaceutically active substances, and as required, an ultraviolet abosorbant in an amount of 0.01 to 5 wt.%, an antioxidation agent in an amount of 0.01 to 5 wt.%, and an antiseptic in an amount of 0.01 to 5 wt.% are combined with any of trimethyl citrate, trietyl citrate, triisopropyl citrate and tributyl citrate which are features of the present invention in an amount of up to 30 wt.%, low-carbon alcohol such as ethanol or isopropanol) in an amount of 10 to 40 wt.%, a water-soluble polyhydric alcohol in an amount of 10 to 89 wt.%, water in an amount of 5 to 40 wt.%, an ionic non-saturated monomer in an amount of 1 to 40 wt.%, methacrylate in an amount of 2 to 30 wt.%, and a cross-linking agent in an amount of 0.05 to 1 wt.%, and the patch drug of the present invention is available through polymerization cross-linking in the presence of a polymerization initiator. The example of prescription and the example of manufacture as presented above are only examples, and the patch drug of the present invention may be available also with a similar prescription.

## PREFERRED EMBODIMENTS OF THE INVENTION

Now, the structure and the effects of the present invention are described below by means of examples. It is needless to mention that the present invention is not limited by these examples.

First, as Example A, a composition comprising trialkyl citrate, low-carbon alcohol and water, and a preparation in which this composition was combined with pharmaceutically active substances are described.

[EXAMPLE A]

EXAMPLES 1-A to 2-A

(COMPARATIVE EXAMPLES 1-A to 2-A)

Comparative test 1-A

As shown in Table 1-A, compositions were prepared for comparison purposes with the composition of the present invention and skin primary irritation thereof was evaluated in accordance with the Draize method.

As is evident from the results shown in Table 1-A, addition of trialkyl citrate clearly brings about a decrease in skin primary irritation attributable to the presence of ethanol.

Table 1-A

| Test No. | Composition | P I I |
|---|---|---|
| Comparative Example 1-A | Water/ethanol/triethyl citrate = 60/40/0 | 1.18 |
| Example 1-A | Water/ethanol/triethyl citrate = 50/40/10 | 0.31 |
| Comparative Example 2-A | Water/ethanol/triethyl citrate = 50/50/0 | 1.56 |
| Example 2-A | Water/ethanol/triethyl citrate = 40/50/10 | 0.44 |

Comparative Test 2-A

For each of the solution and water of the compositions of the above-mentioned Example 1-A and Comparative Example 1-A, skin penetration of the active Components was measured in vitro on skin taken from hairless mice, with the use of morphine hydrochloride suspension. Values of skin penetration of the medications were compared in terms of the maximum Flux (maximum penetration rate in $\mu g/cm^2/hr$) during a period of up to the eighth hour.

The results are as shown in Table 2-A: skin penetration of medication is almost equally accelerated in the Example 1-A and the Comparative Example 1-A. It is however clear from the results of the above-mentioned comparative test 1-A that skin irritation is largely reduced in the Example 1-A.

Table 2-A

| Solution | Max. Flux up to 8th hr. | Absorptrion accelerating rate |
|---|---|---|
| Water | 20.1 | 1 |
| Example 1-A | 198.3 | 9.9 |
| Comparative Example 1-A | 214.8 | 10.7 |

EXAMPLE 3-A

(Comparative Example 3-A)

A solution having the composition of the Example 3-A and one having the composition of the Comparative Example 3-A were prepared, and for each solution, skin penetration of effective components was measured in vitro on skin taken from hairless mice with the medications shown in Table 3, and at the same time, a skin irritation test of the solutions was carried out in accordance with the Draize method. Skin penetration of medications was compared in terms of the maximum Flux (maximum penetration rate in $\mu g/cm^2/hr$) for a period of up to the eighth hour, and skin irritation, in PII (skin irritation index).

(Example 3-A: Composition)

Water/isopropanol/triethyl citrate/azacycloalkane derivative = 67/20/20/3 (W/W)

(Comparative Example 3-A: Composition)

Water/isopropanol/azacycloalkane derivative = 77/20/3 (W/W)

The results are as shown in Table 3-A: while there is no remarkable difference in skin penetration of medication between the two cases, skin irritation considerably decreases in the solution of the Example 3-A and this is considered attributable, to the absorption accelerator (azacycloalkane derivative in this case).

7

Table 3-A

| Medication | Composition | Max. Flux to 8th hr. | PII |
|---|---|---|---|
| Pridinole mesilate | Example 3-A | 835.8 | 0.45 |
| | Comparative Example 3-A | 830.0 | 1.33 |
| Furnalidine hydrochloride | Example 3-A | 87.0 | 0.25 |
| | Comparative Example 3-A | 92.0 | 1.00 |
| Chronidine hydrochloride | Example 3-A | 5253.0 | 0.30 |
| | Comparative Example 3-A | 5300.0 | 1.34 |
| Betalistine mesilate | Example 3-A | 102.0 | 0.42 |
| | Comparative Example 3-A | 105.3 | 1.45 |
| Ketoprophene | Example 3-A | 102.0 | 0.38 |
| | Comparative Example 3-A | 105.5 | 1.38 |
| Meperidine | Example 3-A | 3051.0 | 0.37 |
| | Comparative Example 3-A | 3100.0 | 1.18 |
| * Average over n = 6. | | | |

Examples 4-A to 12-A

(Comparative Examples 4-A to 12-A)

Solutions of the compositions shown in Table 4-A were prepared, and for each solution, skin penetration of effective components was measured in vitro on skin taken from hairless mice with the use of morphine hydrochloride suspension, and at the same time, skin irritation of solution was tested in accordance with the Driaze method.

The results are as shown in Table 4-A. Skin penetration of medication was compared in terms of the maximum Flux (maximum penetration rate) by a penetration test carried out for eight hours. Simultaneous use of trialkyl citrate (triethyl citrate in this case) and the absorption accelerator is found to permit achievement of a skin penetration equal or even superior to the conventional one and a skin irritation further lower than the conventional one. Decrcase in penetration rate is observed in some cases. This is because the ratio of combination of triethyl citrate is constant in this Example. Even in this case, the decrease in skin irritation is even far larger than the decrease in penetration rate.

Table 4-A

| Test No. | Absorption accelerator 5% (W/W) | Composition | Max. Flux to 8th hr. ($\mu$g.cm$^2$/hr) | PII |
|---|---|---|---|---|
| Comparative Example 4-A<br>Example 4-A | Azacycloalkane derivative | Water/IPA = 7/3 (V/V)<br>Water/IPA/TC = 6/3/1 (V/V) | 5.450<br>4.130 | 4.65<br>1.30 |
| Comparative Example 5-A<br>Example 5-A | E-chloro-m-cresole | Water/IPA = 7/3 (V/V)<br>Water/IPA/TC = 6/3/1 (V/V) | 3.423<br>3.162 | 1.70<br>0.45 |
| Comparative Example 6-A<br>Example 6-A | Butylhydroxy anisole | Water/IPA = 7/3 (V/V)<br>Water/IPA/TC = 6/3/1 (V/V) | 4.716<br>1.428 | 1.00<br>0.05 |
| Comparative Example 7-A<br>Example 7-A | Thymol | Water/IPA = 7/3 (V/V)<br>Water/IPA/TC = 6/3/1 (V/V) | 6.459<br>5.653 | 0.60<br>0.00 |
| Comparative Example 8-A<br>Example 8-A | Oleic acid | Water/IPA = 7/3 (V/V)<br>Water/IPA/TC = 6/3/1 (V/V) | 3.088<br>2.877 | 0.50<br>0.25 |
| Comparative Example 9-A<br>Example 9-A | d-limonene | Water/IPA = 7/3 (V/V)<br>Water/IPA/TC = 6/3/1 (V/V) | 1.018<br>1.451 | 0.40<br>0.20 |
| Comparative Example 10-A<br>Example 10-A | n-capric acid | Water/IPA = 7/3 (V/V)<br>Water/IPA/TC = 6/3/1 (V/V) | 5.587<br>5.664 | 0.35<br>0.30 |
| Comparative Example 11-A<br>Example 11-A | 1-menthol | Water/IPA = 7/3 (V/V)<br>Water/IPA/TC = 6/3/1 (V/V) | 3.705<br>2.804 | 0.05<br>0.00 |
| Comparative Example 12-A<br>Example 12-A | Geraniol | Water/IPA = 7/3 (V/V)<br>Water/IPA/TC = 6/3/1 (V/V) | 5.402<br>6.201 | 0.05<br>0.00 |

Example 13-A to 17-A

(Comparative Examples 13-A to 17-A)

For the following compositions of Examples 13-A to 17-A and Comparative Examples 13-A to 17-A, the following tests were carried out.

More specifically, the back of a Japanese white rabbit having a body weight of approximately 3 kg was shaved, and samples of the Example 13 and the Comparative Example 13 (containing 30 mg morphine hydrochloride) were applied to the shaved skin of the back. The applied surface was covered with filter paper, which was then fixed with a tape. Blood was sampled 0, 1, 1, 3 and 5 hours after the application to measure the morphine content. For the Examples 13-A to 17-A and the Comparative Examples 13-A to 17-A, skin irritation was measured in accordance with the Draize method.

(Example 13-A: Gel-form drug)

| | |
|---|---|
| Morphine hydrochloride | 5 |
| Azacycloalkane derivative | 3 |
| Carboxyvinyl polymer | 1 |
| Ethanol | 20 |
| Propylene glycol | 10 |
| Triethyl citrate | 20 |
| Purified water | 39.4 |
| 2-hydroxy-4-methoxybenzophenone | 0.5 |
| Diisopropanol amine | 1.1 |
| Total | 100 (wt.%) |

So as to give the above-mentioned composition, 1 part carboxyvinyl polymer was swelled with 23 parts water, and a mixed solution of 20 parts ethanol, 10 parts propylene glycol, 20 parts triethyl citrate, 5 parts morphine hydrochloride, and 6.5 parts 2-hydroxy-4-methoxybenzophenone was added and stirred. Then, 3 parts, azacycloalkane derivative and 1.1 parts diisopropanol amine dissolved in 16.4 parts purified water were added, and the entire mixture was sufficiently stirred to give a uniform mixture, thus resulting in a gel-form drug.

(Comparative Example 13-A: Gel-form drug)

A gel-form drug was obtained in the same manner as in the Example 13-A except that triethyl citrate was replaced with ethanol.

(Example 14-A: Liniment drug)

| | |
|---|---|
| Methyl salicylate | 2 |
| 1-menthol | 5 |
| Ethanol | 25 |
| Triethyl citrate | 27 |
| Isopropyl myristate | 5 |
| Purified water | 36 |
| Total | 100 (wt.%) |

The components were mixed so as to give the above-listed composition, and a liniment drug was obtained.

(Comparative Example 14-A: Liniment drug)

A liniment drug was obtained in the same manner as in the Example 14-A except that triethyl citrate was replaced with ethanol.

(Example 15-A: Gel-cream drug)

| | |
|---|---|
| Silver sulfur diazine | 5 |
| Geraniol | 3 |
| White vaseline | 5 |
| Cetanol | 4 |
| Liquid paraffin | 3 |
| Triethyl citrate | 15 |
| Paraoxymethyl benzoate | 0.2 |
| Carboxyvinyl polymer | 2 |
| Diisopropanol amine | 1 |
| Paraoxyethylene paraoxypropylene cetyl ether | 2 |
| Isopropanol | 20 |
| Purified water | 39.8 |
| Total | 100 (wt.%) |

So as to give the above-mentioned composition, white vaseline, cetanol, liquid paraffin, triethyl citrate, geraniol, paraoxyethylene paraoxypropylene cetyl ether were mixed and heated for dissolution, and then paraoxymethyl benzoate, isopropanol and silver sulfur diazine dissolved and dispersed in previously heated purified water were added. The resultant mixture was cooled to 50°C, added with carboxyvinyl polymer, and after sufficient dispersion, added with diisopropanol amine to adjust pH to 4 to 8, thus giving a gel-cream drug.

(Comparative Example 15-A: Gel-cream drug)

A gel-cream drug was obtained in the same manner as in the Example 15-A except that triethyl citrate was replaced with purified water.

(Example 16-A: Reservoir type patch drug)

A laminated drug was prepared by achieving a configuration comprising:
1) Support: a polyester film,
2) Medication storing layer: composition of Example 13
3) Microporous membrane: duraguard
4) Adherent layer: acrylic adhesive (surrounding the support),
and applying the peeloff papaer to the surface of the microporous adherent layer.

(Comparative Example 16-A: Reservoir type patch drug)

A reservoir type patch drug was obtained in the same manner as in the Example 13-A except that the medication storing layer was replaced with the composition of the Comparative Example 13-A.

(Example 17-A: Patch drug)

First, 15 parts 1-hydroxycyclohexylphenylketone were dissolved in 2,400 parts 2-hydroxyethyl methacrylate, and 35 parts tetraethylene glycol dimethaorylate were added and dissolved. Separately, 1,900 parts 5% salt water and 600 parts sulfopropyl methacrylate potassium salt were heated and dissolved, and furthermore, 2,000 parts glycerine, 2,000 parts isopropanol, 1,100 parts triethyl citrate, 110 parts ketoprophene, and 300 parts capric acid were added to give a uniform solution. These solutions were mixed and bubbles were removed to obtain a uniform solution. The resultant solution was poured into a thickness of 300 $\mu$m on a PET film kept horizontal, which had a thickness of 50 $\mu$m and was provided with a frame to prevent the solution, from flowing out. This was placed 15 cm below a 30 W chemical lamp while keeping it in a nitrogen atmosphere, and after irradiation of ultraviolet ray for five minutes, covered with a PET film, the hardened surface of which was subjected to a stripping treatment, thus giving a patch drug.

(Comparative Example 17-A: Patch drug)

A patch drug was obtained in the same manner as in the Example 17-A except that triethylene citrate was replaced with glycerine.

The results of tests of the above-mentioned compositions are as shown in Tables 5-A and 6-A. No significant difference is observed in the transfer of morphine hydrochloride into blood between the Examples and the Comparative Examples, but irritation to skin is significantly reduced in the Examples.

Table 5-A

| Blood sampled after the lapse of : | Morphine concentration in blood (ng/ml) | |
|---|---|---|
| | Example 13-A | Comparative Example 13-A |
| 0 hr. | ND | ND |
| 1 hr. | 98.5 | 104.8 |
| 2 hrs. | 298.3 | 310.5 |
| 3 hrs. | 300.4 | 315.8 |
| 6 hrs. | 297.5 | 299.8 |
| * ND: The value is under the measurement limit. Figures are averages over n = 3. | | |

EP 0 535 237 A1

Table 6-A

| Test No. | PII |
|---|---|
| Example 13-A | 0.13 |
| Comparative Example 13-A | 1.35 |
| Example 14-A | 0.00 |
| Comparative Example 14-A | 0.10 |
| Example 15-A | 0.05 |
| Comparative Example 15-A | 0.15 |
| Example 16-A | 0.36 |
| Comparative Example 16-A | 1.95 |
| Example 17-A | 0.35 |
| Comparative Example 17-A | 0.75 |
| * Average over n = 5. | |

Now, under the heading of the Examples B, a composition comprising trialkyl citrate, low-carbon alcohol, water and a terpene compound serving as an absorption accelerator and a preparation accomplished by combining a pharmaceutically active substance with this composition will be described below.

[EXAMPLES B]

First, the evaluation test is as follows.

[Skin penetration test of medication on skin taken from hairless mice]

Triethyl citrate, isopropanol and water are mixed in the following composition, and various terpene compounds as abserption accelerators are added each in an amount of 5 wt.% relative to the mixture as 95 wt.%. Then, medications (ketoprophene, chroridine hydrochloride, morphine hydrochloride and eperizone hydrochloride) are added to a level at which the final composition is saturated at 37°C.

The resultant drug/percutaneously absorbed preparation was subjected to a skin penetration test with skin cut from the abdomen of hairless mice. More specifically, the effect of addition of various monoterpene compounds on percutaneous absorption of medications was investigated. For evaluation purposes, the maximum skin penetration rate of medications for a period of eight hours after the start of test was determined for each additive as to each medication, and the acceleration rate for control was defined as the penetration acceleration rate to compare values of such rate.

| ● Sample prescription: | |
|---|---|
| (1) Triethyl citrate/isopropanol/water = 10/30/60 (% V/V)<br>(2) Various monoterpene compounds<br>Total : | 94 wt.%<br>5 wt.%<br>100 wt.% |

| ● Control prescription: | |
|---|---|
| (1) Triethyl citrate/isopropanol/water = 10/30/60 (% V/V) | 100 wt.% |

12

EP 0 535 237 A1

| ● Comparative prescription: | |
|---|---|
| (1) Isopropanol/water = 30/70 (% V/V) | 95 wt.% |
| (2) Various monoterpene compounds | 5 wt.% |
| Total : | 100 wt.% |

[Skin primary irritation test of percutaneously administered basal drug containing various monoterpene compounds]

A drug containing various medications and additives in an amount of 100 μl was caused to permeate into a Torii's patch plaster (small) to evaluate skin irritation in accordance with the Draize method. For each medication, five Japanese white rabbit were shaved with an electric hair-cutter and a shaver for application of drugs. The same prescription as that for the above-mentioned skin penetration test was used for the sample composition.

The skin penetration accelerating rate and skin irritation index were evaluated by the methods as described above. The results of tests are shown together with those of the Comparative Examples.

Examples 1-B to 4-B

(Comparative Examples 1-B to 4-B)

This test was to evaluate skin irritation and skin penetration for each prescription when using ketoprophene as the medication.

The results as shown in Tables 1-B and 2-B were obtained. In the case of the Examples of the present invention shown in Table 1-B, excellent results were obtained in terms of both penetration accelerating rate and skin irritation index.

Table 1-B

| Example No. | Medication | Monoterpene compound | Penetration accelerating rate | Skin irritation index (PII-value) |
|---|---|---|---|---|
| 1 - B | Ketoprophene | Geraniol | 44 | 0.56 |
| 2 - B | Ketoprophene | Borneorol | 40 | 0.15 |
| 3 - B | Ketoprophene | Terpeneol | 34 | 0.16 |
| 4 - B | Ketoprophene | L-menthol | 20 | 0.34 |
| Control | Ketoprophene | None | 1 | 0.1 |

Table 2-B

| Comparative Example No. | Medication | Monoterpene compound | Penetration accelerating rate | Skin irritation index (PII-value) |
|---|---|---|---|---|
| 1 - B | Ketoprophene | Geraniol | 43 | 1.43 |
| 2 - B | Ketoprophene | Borneorol | 41 | 1.22 |
| 3 - B | Ketoprophene | Terpeneol | 35 | 0.87 |
| 4 - B | Ketoprophene | L-menthol | 23 | 0.98 |
| * Penetration accelerating rate: The value obtained by dividing the maximum penetration rate for a period of up to the eighth hour after the start of test by the maximum penetration rate available in the reference example. | | | | |

13

Examples 5-B to 8-b

(Comparative Examples 5-B to 8-B)

This test was to evaluate skin irritation and skin penetration for each prescription when using eporizone hydrochloride as the medication.

The results as shown in Tables 3-B and 4-B were obtained. Excellent results were obtained in the Examples of the present invention.

Table 3-B

| Example No. | Medication | Monoterpene compound | Penetrating accelerating rate | Skin irritation index (PII-value) |
|---|---|---|---|---|
| 5 - B | Eperizone hydrochloride | Geraniol | 49 | 0.32 |
| 6 - B | Eperizone hydrochloride | Thymol | 45 | 0.20 |
| 7 - B | Eperizone hydrochloride | L-menthol | 38 | 0.13 |
| 8 - B | Eperizone hydrochloride | d - ( + ) -limonene | 13 | 0.23 |
| Control | Eperizone hydrochloride | None | 1 | 0.14 |

Table 4-B

| Comparative Example No. | Medication | Monoterpene compound | Penetration accelerating rate | Skin irritation index (PII-value) |
|---|---|---|---|---|
| 5 - B | Eperizone hydrochloride | Geraniol | 50 | 1.01 |
| 6 - B | Eperizone hydrochloride | Thymol | 46 | 0.95 |
| 7 - B | Eperizone hydrochloride | L-menthol | 40 | 0.87 |
| 8 - B | Eperizone hydrochloride | d - ( + ) -limonene | 16 | 1.21 |

Examples 9-B to 12-B

(Comparative Examples 9-B to 12-B)

This test was to evaluate skin irritation and skin penetration for each prescription when using morphine hydrochloride as the medication.

The results as shown in Tables 5-B and 6-B were obtained. Excellent results were obtained in the Examples of the present invention.

14

EP 0 535 237 A1

Table 5-B

| Example No. | Medication | Monoterpene compound | Penetration accelerating rate | Skin irritation index (PII-value) |
|---|---|---|---|---|
| 9 - B | Morphine hydrochloride | Geraniol | 124 | 0.34 |
| 10 - B | Morphine hydrochloride | Terpineol | 97 | 0.16 |
| 11 - B | Morphine hydrochloride | Thymol | 113 | 0.29 |
| 12 - B | Morphine hydrochloride | L-menthol | 76 | 0.15 |
| Control | Morphine | None | 1 | 0.17 |

Table 6-B

| Comparative Example No. | Medication | Monoterpene compound | Penetration accelerating rate | Skin irritation index (PII-value) |
|---|---|---|---|---|
| 9 - B | Morphine hydrochloride | Geraniol | 125 | 1.56 |
| 10 - B | Morphine hydrochloride | Terpineol | 124 | 1.31 |
| 11 - B | Morphine hydrochloride | Thymol | 123 | 1.02 |
| 12 - B | Morphine hydrochloride | L-menthol | 94 | 1.16 |

Examples 13-B to 16-B

(Comparative Examples 13-B to 16-B)

This test was to evaluate skin irritation and skin penetration for each prescription when using chronidine hydrochloride as the medication.

The results as shown in Tables 7-B and 8-B were obtained. Excellent results were obtained in the Examples of the present invention in terms of alleviation of skin irritation and there is no degradation of skin penstration.

Table 7-B

| Example No. | Medication | Monoterpene compound | Penetration accelerating rate | Skin irritation index (PII-value) |
|---|---|---|---|---|
| 13 - B | Chronidine hydrochloride | Borneorol | 91 | 0.11 |
| 14 - B | Chronidine hydrochloride | Terpineol | 77 | 0.13 |
| 15 - B | Chronidine hydrochloride | d, 1-camphor | 6 | 0.05 |
| 16 - D | Chronidine hydrochloride | Eugenol | 17 | 0.05 |
| Control | Chronidine hydrochloride | None | 1 | 0.05 |

15

Table 8-B

| Comparative Example No. | Medication | Monoterpene compound | Penetration accelerating rate | Skin irritation index (PII-value) |
|---|---|---|---|---|
| 13 - B | Chronidine hydrochloride | Borneorol | 93 | 0.89 |
| 14 - B | Chronidine hydrochloride | Terpineol | 77 | 0.97 |
| 15 - B | Chronidine hydrochloride | d, 1-camphor | 7 | 1.05 |
| 16 - B | Chronidine hydrochloride | Eugenol | 17 | 0.88 |

According to the evaluation in terms of skin irritation index as outlined in the above description of the Examples 1-B to 16-B, all the mixtures containing both triethyl citrate and monoterpene compounds were substantially non-irritating to skin except for the Comparative Examples in which a slight irritation was observed for all the medications. It has furthermore become clear that a percutaneous absorption far superior to that of the control not containing monoterpene compounds is demonstrated.

Example 17-B

(Comparative Example 17-B)

Gel-form, gel-cream, liniment, reservoir-type patch and patch drugs were prepared in accordance with the respective prescriptions shown below on the basis of the medication administering composition of the present invention, and simultaneously, comparative drugs were prepared in compliance with respective comparative prescriptions. For each of the thus prepared drugs, skin penetration of medication and skin irritation were evaluated in vitro by means of rabbit skin.

(Gel-form prescription)

| | |
|---|---|
| Morphine hydrochloride | 5 |
| L-menthol | 3 |
| Carboxyvinyl polymer | 1 |
| Ethanol | 20 |
| Propylene glycol | 15 |
| Triethyl citrate | 15 |
| Purified water | 39.4 |
| 2-hydroxy-4-methoxybenzophenone | 0.5 |
| Diisopropanol amine | 1.1 |
| Total | 100 (wt.%) |

So as to achieve the above-mentioned composition, carboxyvinyl polymer in an amount of 1 part was swelled with purified water in an amount of 23 parts, and a solution containing in mixture 20 parts ethanol, 15 parts propylene glycol, 15 parts triethyl citrate, 5 parts morphine hydrochloride, and 0.5 parts 2-hydroxy-4-methoxybenzophenone was added and stirred. Then, 3 parts L-menthol and 1.1 parts diisopropanol amine dissolved in 16.4 parts purified water were added, and the entire mixture was sufficiently stirred to achieve uniformity thereof, thus giving a gel-form drug.

(Comparative prescription for gel-form drug)

A comparative drug was prepared in the same manner as above except that triethyl citrate was replaced with ethanol.

(Gel-cream prescription)

| | |
|---|---|
| Morphine hydrochloride | 5 |
| Terpineol | 3 |
| White vaseline | 5 |
| Cetanol | 4 |
| Liquid paraffin | 3 |
| Triethyl citrate | 15 |
| Paraoxymethyl benzoate | 0.2 |
| Carboxyvinyl polymer | 1 |
| Diisopropanol amine | 1 |
| Paraoxyethylene paraoxypropylene cetyl ether | 2 |
| Purified water | 60.8 |
| Total | 100 (wt.%) |

So as to achieve the above composition, white vaseline, cetanol, liquid paraffin, triethyl citrate, terpineol, and paraoxyethylene paraoxypropylene cetyl ether were mixed, heated and dissolved, and then, paraoxymethyl benzoate and methyl salicylate dissolved and dispersed in previously heated purified water were added. The resultant mixture was then cooled to 50°C, and after addition and sufficient dispersion of carboxyvinyl polymer, added with diisopropanol amine to adjust pH to 4 to 8 thus giving a gel-cream drug.

(Comparative prescription for gel-cream drug)

A comparative gel-cream drug was prepared in the same manner as above except that triethyl citrate in the above-mentioned gel-cream prescription was replaced with purified water.

(Prescription for liniment drug)

| | |
|---|---|
| Morphine hydrochloride | 5 |
| d-( + )-limonene | 5 |
| Ethanol | 25 |
| Triethyl citrate | 30 |
| Triethyl myristate | 5 |
| Purified water | 30 |
| Total | 100 (wt.%) |

The composition as listed above was mixed as prescribed and a liniment drug was obtained.

(Comparative prescription for liniment drug)

A comparative liniment drug was prepared in the same manner as above except that triethyl citrate was replaced with ethanol.

(Reservoir type patch drug)

The following components were combined:
1) Support: polyester film,
2) Medication storing layer: the composition used in the above-mentioned gel-form drug,
3) Microporous membrane: duraguard,
4) Adherent layer: acrylic adhesive (adherent layer surrounding the support),
and a peeloff paper was applied to the surfaces of the microporous membrane and the adherent layer to prepare a lamination.

(Comparative Example of reservoir type patch drug)

A comparative reservoir type patch drug was prepared in the same manner as above except that the contents of the medication storing layer were replaced with the composition of the comparative gel-form drug.

(Patch drug)

First, 15 parts 1-hydroxycyclohexyl phenylketone were dissolved in 2,400 parts 2-hycroxyethyl methacylate, and 35 parts tetraethylene glycol dimethacrylate were added and dissolved. Separately, 1,900 parts 5% salt water and 600 parts sulfopropyl methacrylate potassium salt were added and dissolved. Then, 2,000 parts glycerine, 2,000 parts isopropanol, 1,100 parts triethyl citrate, 110 parts morphine hydrochloride, and 300 parts thymol were added to obtain a uniform solution. These solutions were mixed and bubbles were removed to obtain a uniform solution. The resultant solution was poured into a thickness of 300 $\mu$m on a PET film kept horizontal, which had a thickness of 50 $\mu$m and was provided with a frame to prevent the solution from flowing out. This was placed 15 cm below a 30 W chemical lamp while keeping it in a nitrogen atmosphere, and after irradiation of ultraviolet ray for five minutes, covered with a PET film, the hardened surface of which was subjected to a stripping treatment, thus giving a patch drug.

(Comparative patch drug)

A comparative patch drug was prepared in the same manner as above except that triethyl citrate was replaced with glycerine.

(Evaluation test)

The following test was carried out for the drugs prepared in accordance with the above mentioned prescriptions for gel-cream drugs.

More specifically, the back of a Japanese white rabbit having a body weight of approximately 3 kg was shaved, and samples of the comparative Example were applied to the shaved skin of the back. The applied surface was covered with filter paper, which was then fixed with a tape. Blood was sampled 0, 1, 2, 3 and 6 hours after the application to measure the morphine content in blood. The results are shown in Table 9-B.

Skin irritation of the drugs of the prescriptions for ge-form, gel-cream, liniment, patch and reservoir type patch drugs of morphine hydrochloride was evaluated, including the comparative ones, in accordance with the Draize method. These drugs were prepared in compliance with the above-mentioned Examples and Comparative Examples to apply comparative tests. The results are shown in Table 10-B.

Table 9-B

| Morphine concentration in blood (ng/ml) | | |
|---|---|---|
| Blood sampled after the lapse of: | Example 13-B | Comparative Example 13-B |
| | ND | ND |
| 1 hr. | 27.5 | 32.0 |
| 2 hrs. | 178.3 | 161.5 |
| 3 hrs. | 214.5 | 205.4 |
| 6 hrs. | 184.2 | 178.9 |
| * ND: under the measurement limit Figures are averages over n = 3. | | |

18

Table 10-B

| Prescription of the invention | Gel | Gel-cream | Liniment | Reservoir type patch | Patch |
|---|---|---|---|---|---|
| PII-value | 0.14 | 0.33 | 0.15 | 0.20 | 0.45 |
| Comparative prescription | Gel | Gel-cream | Liniment | Reservoir type patch | Patch |
| PII-value | 1.05 | 1.20 | 1.36 | 1.10 | 1.40 |
| * Figures are averages over n = 5. | | | | | |

As is clear from Table 9-B, no significant difference is observed in the transfer of morphine from the gel drug into blood between the Example and the Comparative Example, and it is suggested that percutaneous absorption is kept even by the combination of triethyl citrate. From Table 10-B, there was found a tendency of uniform decrease in skin irritation in the gel, gel-cream, liniment, reservoir type patch and patch drugs containing triethyl citrate as compared with the comparative cases.

Now, as the Examples C, the composition comprising trialkyl citrate, low-carbon alcohol and water, and an absorption accelerator selected from fatty acids, fatty acid alcohols, fatty acid esters, and phenol derivatives and the drug comprising this composition combined with pharmaceutically active substances are described below.

[EXAMPLE C]

Example 1-C

(Skin temporary irritation and percutaneous absoption accelerating function of the composition)

Skin temporary irritation of the composition of the present invention and its accelerating function of percutaneous absorption of medications were evaluated. The sample solution was prepared by adding physiologically active substances (pridinole mecilate, furnaridine hydrochloride, chronidine hydrochloride, betahistine mecilate, ketoprohene, aspirine and meperidine) in an amount of saturation to a solution prepared by adding various absorption accelerators in an amount of 5% to water, isopropanol and triethyl citrate in amounts of 65%, 30% and 10%, respectively, as shown in Tables 1 to 4, and dissolving same at 37°C.

The in vitro penetration of medications was evaluated by holding skin taken from the side abdomen of a female hairless mouse in a horizontal type 2-chamber cell, filling the cell on the horny substance layer side with 2.7 ml sample solution, filling the corium side cell with physiological saline solution, and determining the amount of penetrating medication with time at 37°C from the supply to receiving sides. Quantitative determination of medication was conducted by means of an HPLC, and the maximum penetration rate up to the eighth hour was compared. Simultaneously with this, skin irritation of the medication in the form of saturated solution was measured in accordance with the Draize method. Samples not containing triethyl citrate in the composition were prepared, and values of skin irritation index (PII-values) thus determined were compared. The results are shown in Tables 1-C to 4-C.

Table 1-C·

| Test No. | Medication | Absorption accelerator | Penetration accelerating rate [*] | Irritation reducing rate [**] |
|---|---|---|---|---|
| 1 - C | Pridinol | Oleic acid | 108 | 0.50 |
| 2 - C | mecilate, | Oleil alcohol | 124 | 0.57 |
| 3 - C | | Myristil alcohol | 40 | 0.65 |
| 4 - C | | Cetyl palminate | 44 | 1.00 |
| 5 - C | | Diethyl sebacate | 32 | 1.00 |
| 6 - C | | Capric acid | 116 | 0.86 |
| 7 - C | | Oleil oleate | 84 | 0.55 |
| 8 - C | Chronidine | Capric acid | 185 | 0.16 |
| 9 - C | hydro- | Salicylic acid | 62 | 1.00 |
| 10 - C | chloride | Oleil alcohol | 123 | 0.57 |
| 11 - C | | Myristil alcohol | 92 | 0.65 |
| 12 - C | | Butylhydroxy anisole | 191 | 0.05 |
| 13 - C | | Chlor creosole | 168 | 0.26 |
| 14 - C | | Linolic acid | 70 | 0.12 |
| 15 - C | | Polyethylene glycol monostearate | 81 | 0.48 |

Table 2-C

| Test No. | Medication | Absorption accelerator | Penetration accelerating rate * | Irritation reducing rate ** |
|---|---|---|---|---|
| 16 - C | Furnadine | Capric acid | 11 | 0.86 |
| 17 - C | hydro- | Caproic acid | 6 | 0.50 |
| 18 - C | chloride | linolic acid | 6 | 0.12 |
| 19 - C | | Oleic acid | 5 | 0.50 |
| 20 - C | | Chlor cresole | 7 | 0.26 |
| 21 - C | | Butylhydroxy anisole | 2 | 0.05 |
| 22 - C | | Methyl cinnamate | 1 | 0.05 |
| 23 - C | | Diethyl sebacate | 1 | 1.00 |
| 24 - C | Betahistine | Chlor cresole | 173 | |
| 25 - C | mecilate | Capric acid | 171 | |
| 26 - C | | Methyl cinnamate | 75 | |
| 27 - C | | Cetyl palminate | 62 | |
| 28 - C | | Myristic acid | 53 | |
| 29 - C | | Oleic acid | 80 | |
| 30 - C | | Sorbitan Sesquioleate | 29 | |
| 31 - C | | Methyl laurate | 21 | |

Table 3-C

| Test No. | Medication | Absorption accelerator | Penetration accelerating rate * | Irritation reducing rate ** |
|----------|------------|------------------------|--------------------------------|-----------------------------|
| 32 - C | Ketoprophene | Capric acid | 46 | 0.86 |
| 33 - C | | Linolic acid | 40 | 0.12 |
| 34 - C | | Salicylic acid | 17 | 1.00 |
| 35 - C | | Polyethylene glycol Monostearate | 23 | 0.48 |
| 36 - C | | Oleil alcohol | 28 | 0.57 |
| 37 - C | | Myristic acid | 19 | 0.52 |
| 38 - C | Aspirine | Capric acid | 38 | 0.16 |
| 39 - C | | Chlor cresole | 25 | 0.26 |
| 40 - C | | Butylhydroxy anisole | 33 | 0.05 |
| 41 - C | | Methyl cinnamate | 18 | 0.05 |
| 42 - C | | Diethyl sebacate | 11 | 1.00 |
| 43 - C | | Sorbitan sesquioleate | 13 | 0.80 |

22

Table 4-C

| Test No. | Medication | Absorption accelerator | Penetration accelerating rate * | Irritation reducing rate ** |
|---|---|---|---|---|
| 44-C | Meperidine | Capric acid | 99 | 0.16 |
| 45-C | | Caproic acid | 95 | 0.50 |
| 46-C | | Oleic acid | 82 | 0.50 |
| 47-C | | Myristil alcohol | 71 | 0.65 |
| 48-C | | Sorbitan sesquioleate | 25 | 0.80 |
| 49-C | | Methyl laurate | 21 | 0.75 |

*: The value calculable by dividing the maximum penetration rate of medications in various samples by the maximum penetration rate in a water/isopropanol (70/30%) solution not containing an absorption accelerator or triethyl citrate.

**: The value calculable by dividing the PII-value of each sample solution by the PII-value of a comparative example not containin triethyl citrate.

As shown in Tables 1-C to 4-C, the absorption accelerating effect in the case where an absorption accelerator and triethyl citrate are added is very remarkable as compared with the skin penetration available when a medication is dissolved in a two-component mixed solution of water and isopropanol, so that it is now possible to improve absorbability to an extent sufficient to make even a medication having a low percutaneous absorption display its pharmaceutical activity. At the same time, combination of triethyl citrate with the sample solution permits considerable alleviation of the high skin irritation of the absorption accelerator, and it is now possible to provide a composition for administration posing no practical problem in terms of both skin irritation and percutaneous absorption.

Example 2-C

(Gel-form drug)

| Morphine hydrochloride | 5 |
|---|---|
| Triethyl citrate | 20 |
| Capric acid | 3 |
| Carboxyvinyl polymer | 1 |
| Isopropanol | 20 |
| Propylene glycol | 10 |
| 2-hydroxy-4-methoxybenzophenone | 0.5 |
| Diisopropanol amine | 1.1 |
| Purified water | 39.4 |
| | 100 (wt.%) |

So as to achieve the above-mentioned composition, carboxyvinyl polymer was caused to swell with half an amount of purified water, added to a solution containing dissolved ethanol, propylene glycol, triethyl citrate, capric acid, morphine hydrochlorlde and 2-hydroxy-4-methoxybenzophenone, and the resultant solution was stirred. Then, diisopropanol amine as dissolved in the rest of purified water was added to the solution, and the mixture was sufficiently stirred until overall uniformity was achieved, thus giving a gel-form drug.

Comparative Example 1-C

(Gel-form drug)

A comparative gel-form drug was prepared in the same manner as above except that triethyl citrate in the Example 2-C was replaced with purified water.

Reference Example 1-C

(Gel-form drug)

Further another gel-form drug was prepared in the same manner as in the Example 2-C except that triethyl citrate and capric acid were replaced with purified water.

Example 3-C

(Liniment drug)

| Morphine hydrochloride | 1 |
|---|---|
| Triethyl citrate | 30 |
| Methyl cinnamate | 4 |
| L-menthol | 5 |
| Ethanol | 25 |
| Isopropyl myrisate | 5 |
| Purified water | 30 |
| | 100 (wt.%) |

So as to achieve the above-mentioned composition, morphine hydrochloride, menthol, ethanol, trietyl citrate, methyl cinnamate, isopropyl myrisate and purified water were mixed in prescribed amounts to give a liniment drug.

24

Comparative Example 2-C

(Liniment drug)

A comparative liniment drug was prepared in the same manner as above except that triethyl citrate in the Example 3-C was replaced with purified water.

Example 4-C

(Gel-cream drug)

| | |
|---|---|
| Morphine hydrochloride | 5 |
| Linolic acid | 3 |
| Triethyl citrate | 15 |
| Isopropanol | 20 |
| White vaseline | 5 |
| Cetanol | 4 |
| Liquid paraffin | 3 |
| Paraoxymethyl benzoate | 0.2 |
| Carboxyvinyl polymer | 2 |
| Diisopropanol amine | 1 |
| Paraoxyethylene paraoxypropylene cetyl ether | 2 |
| Purified water | 39.8 |
| | 100 (wt.%) |

So as to achieve the above-mentioned composition, white vaseline, cetanol, liquid paraffin, triethyl citrate, linolic acid and paraoxyethylene paraoxypropylene cetyl ether were mixed, heated and dissolved, and then added with a solution prepared by dissolving and dispersing paraoxymethyl benzoate, isopropanol and morphine hydrochloride in previously heated water. The entire mixture was then cooled to 50°C, and after addition and sufficient dispersion of carboxyvinyl polymer at this temperature, added with diisopropanol amine to adjust pH to 4 to 8, thus giving a gel-cream drug.

Comparative Example 3-C

(Gel-cream drug)

A comparative drug was prepared in the same manner as above except that triethyl citrate in the Example 4-C was replaced with purified water.

Example 5-C

(Reservoir type drug)

A drug known as a reservoir type one was prepared, with a polyester film as the support, by arranging an acrylic adherent layer surrounding the support on the side in contact with skin of a medication storing layer, which contained the composition of the Example 2-C, and a microporous membrane, such as duraguard, as the medication releasing surface at the center, and applying a peeloff paper to the microporous membrane and the adherent layer surface.

Comparative Example 4

(Reservoir type drug)

A comparative drug was prepared in the same manner as above except that the contents of the medication storing layer of the Example 5-C were replaced with the composition of the Comparative Example 1-C.

25

Example 6-C

(Patch drug)

First, 15 parts 1-hydroxycyclohexylphenylketone were dissolved in 2,400 parts 2-hydroxyethyl methacrylate, and 35 parts tetraethylene glycol dimethacrylate were added and dissolved. Separately, 1,900 parts 5% salt water and 600 parts sulfopropyl methacrylate potassium salt were heated and dissolved, and furthermore, 2,000 parts glycerine, 2,000 parts isopropanol, 1,100 parts triethyl citrate, 110 parts morphine hydrochloride, and 300 parts chlor cresole were added to give a uniform solution. These solutions were mixed and bubbles were removed to obtain a uniform solution. The resultant solution was poured into a thickness of 300 $\mu$m on a PET film kept horizontal, which had a thickness of 50 $\mu$m and was provided with a frame to prevent the solution from flowing out. This was placed 15 cm below a 30 W chemical lamp while keeping it in a nitrogen atmosphere, and after irradiation of ultraviolet ray for five minutes, covered with a PET film, the hardened surface of which was subjected to a stripping treatment, thus giving a patch drug.

Comparative Example 5-C

(Patch drug)

A comparative drug was prepared in the same manner as above except that triethyl citrate in the Example 6-C was replaced with liquid paraffin.

Example 7-C

(Evaluation of penetration and irritation)

The following tests were carried out for the above-mentioned Example 2-C, Comparative Example 1-C and Reference Example 1-C.

More specifically, the back of a Japanese White rabbit having a body weight of approximately 3 kg was shaved, and samples of the Example 2-C, the Comparative Example 1-C and the Reference Example 1-C (containing 30 mg morphine hydrochloride) were applied to the shaved skin of the back. The applied surface was covered with filter paper, which was then fixed with a tape. Blood was sampled 0, 1, 2, 3 and 6 hours after the application to measure the morphine content in blood. For the Examples 2-C to 6-C and the Comparative Examples 1-C to 5-C, skin irritation was measured in accordance with the Draize method. The results of these tests are shown in Tables 5-C and 6-C.

Table 5-C

| Blood sampled after the lapse of: | Morphine concentration in blood (ng/ml) | | |
|---|---|---|---|
| | Example 1-C | Comparative Example 1-C | Reference Example 1-C |
| 0 hr. | ND | ND | ND |
| 1 hr. | 284.4 | 296.8 | ND |
| 2 hrs. | 348.3 | 352.1 | ND |
| 3 hrs. | 358.0 | 355.3 | ND |
| 6 hrs. | 354.5 | 361.2 | ND |
| * ND: under the measurement limit<br>Figures are averages over n = 3. | | | |

26

EP 0 535 237 A1

Table 6-C

| Test No. | P I I |
|---|---|
| Example 2-C | 0.20 |
| Comparative Example 1-C | 1.35 |
| Example 3-C | 0.05 |
| Comparative Example 2-C | 0.40 |
| Example 4-C | 0.25 |
| Comparative Example 3-C | 2.15 |
| Example 5-C | 0.20 |
| Comparative Example 4-C | 1.30 |
| Example 6-C | 0.35 |
| Comparative Example 5-C | 1.65 |
| * Averages over n = 5. | |

As shown in Table 5-C, no morphine is detected in blood in the Reference Example 1-C not containing triethyl citrate nor capric acid, and a significant difference is not observed in the transfer of morphine hydrochloride into blood between the Example 2-C and the Comparative Example 1-C. A significant decrease is however observed in skin irritation as shown in Table 6.

Finally, compositions comprising triethyl citrate, low-carbon alcohol, water and an azacyloalkane derivative serving as an absorption accelerator, and drugs prepared by combining these compositions with pharmaceutically active substances are described as Example D.

[EXAMPLE D]

Example 1-D

A composition comprising water/isopropanol/triethyl citrate/1 - [2 - (decilthio) ethyl] azacyclo-pentane-2-on = 67/20/10/3 (W/W) was prepared, and skin primary irritation thereof was evaluated in accordance with the Draize method. The results are shown in Table 1-D. As is clear from the comparison with the Comparative Example, addition of trialkyo citrate clearly rescues skin primary irritation considered attributable to the presence of the absorption accelerator.

Comparative Example D-1

Evaluation was made as to a composition comprising water/isopropanol/1 - [2 - (decylthio) ethyl) azacyclo-pentane-2-on = 77/20/3 (W/W) in the same manner as in the Example 1-D. The results are shown in Table 1-D.

Example 2-D

For a composition comprising water/isopropanol/triethyl citrate = 60/30/10 (V/V): 1-n-dodecylazacycloheptance-2-on = 95 : 5 (W/W), skin primary irritation was evaluated in the same manner as in the Example 1-D. As is clear from the results shown in Table 1-D, addition of trialkyl citrate brings about a decrease in skin primary irritation considered to be caused by the presence of the absorption accelerator.

Comparative Example 2-D

Skin primary irritation was evaluated for a composition comprising water/isopropanol = 70/30 (V/V): 1-n-dodecylazacycloheptance-2-on = 95 : 5 (W/W) in the same manner as in the Example 1-D. The results are shown in Table 1-D.

27

Table 1-D

| Composition | Example 1-D | Comparative Examples 1-d | Example 2-D | Comparative Example 2-d |
|---|---|---|---|---|
| PII | 0.45 | 1.35 | 1.30 | 4.65 |
| * Averages over n = 6. | | | | |

Examples 3-D is 9-D

For solutions having the compositions of the above-mentioned Example 2-D and the Comparative Example 2-D, skin penetration of effective components was measured in vitro on skin taken from hairless mice with the medications shown in the following Table 2-D, and at the same time, a skin irritation test of the solutions was carried out in accordance with the Daize method. Skin penetration of medications was compared in terms of the maximum Flux (maximum penetration rate in $\mu g/cm^2/hr$) for a period of up to the eighth hour, and skin irritation, in terms of PII (skin irritation index).

The results are as shown in Table 2-n: while there is no remarkable difference in skin penetration of medication between the two cases, skin irritation is considerably reduced in the composition of the Example.

Table 2-D

| No. | Medication | Composition | Max. Flux to 8th hr. | PII |
|---|---|---|---|---|
| Example 3-D<br>Comparative Example 3-D | Pridinol mecilate | Example 1-D<br>Comparative Example 1-D | 835.8<br>830.0 | 0.45<br>1.33 |
| Example 4-D<br>Comparative Example 4-D | Furnalidine hydrochloride | Example 1-D<br>Comparative Example 1-D | 87.0<br>92.0 | 0.25<br>1.00 |
| Example 5-D<br>Comparative Example 5-D | Chronidine hydrochloride | Example 1-D<br>Comparative Example 1-D | 5253.0<br>5300.0 | 0.30<br>1.34 |
| Example 6-D<br>Comparative Example 6-D | Betahistine mecilate | Example 1-D<br>Comparative Example 1-D | 102.0<br>105.3 | 0.42<br>1.45 |
| Example 7-D<br>Comparative Example 7-D | Ketoprophene | Example 1-D<br>Comparative Example 1-D | 102.0<br>105.3 | 0.38<br>1.38 |
| Example 8-D<br>Comparative Example 8-D | Aspirine | Example 1-D<br>Comparative Example 1-D | 4306.4<br>4487.8 | 0.22<br>1.15 |
| Example 9-D<br>Comparative Example 9-D | Meperidine | Example 1-D<br>Comparative Example 1-D | 3051.0<br>3100.0 | 0.37<br>1.18 |
| * Averages over n = 6. | | | | |

Example 10-D

| | |
|---|---|
| Morphine hydrochloride | 5 |
| Azacyloalkane derivative | 3 |
| Carboxyvinyl polymer | 1 |
| Ethanol | 20 |
| Propylene glycol | 10 |
| Triethyl citrate | 20 |
| Purified water | 39.4 |
| 2-hydroxy-4-methoxybenzophenone | 0.5 |
| Diisopropanol amine | 1.1 |
| | 100 (wt.%) |

So as to give the above-mentioned composition, 1 part carboxyvinyl polymer was swelled with 23 parts water, and a mixed solution of 20 parts ethanol, 10 parts propylene glycol, 20 parts triethyl citrate, 5 parts morphine hydrochloride, and 6.5 parts 2-hydroxy-4-methoxybenzophenone was added and stirred. Then, 3 parts azacycloakane derivative and 1.1 parts diisopropanol amine dissolved in 16.4 parts purified water were added, and the entire mixture was sufficiently stirred to give a uniform mixture, thus giving a gel-form drug.

For this gel-form drug, together with the following Comparative Example 10-D, the tests as described below were carried out. The back of a Japanese white rabbit having a body weight of about 3 kg was shaved, and the sample (containing 30 mg morphine hydrochloride) were applied to the shaved skin of the back. The applied surface was covered with filter paper, which was then fixed with a tape. Blood was sampled 0, 1, 2, 3 and 6 hours after application to measure the morphine content in blood. For these samples, together with the following Example 11-D to 14-D and the Comparative Example 11-D to 14-D, skin irritation was evaluated in accordance with the Draize method. The results of these tests are as shown in Tables 3-D and 4-D. No significant difference is observed in the transfer of morphine hydrochloride into blood between the Examples and the Comparative Example, but skin irritation is significantly reduced in the Example.

Comparative Example 1--D

A comparative gel-form drug was prepared in the same manner as above except the triethyl citrate was replace with ethanol.

Table 3-D

| Blood sampled after the lapse of: | Morphine concentration in blood (ng/ml) | |
|---|---|---|
| | Example 10-D | Comparative Example 10-D |
| 0 hr. | ND | ND |
| 1 hr. | 98.5 | 104.8 |
| 2 hrs. | 298.3 | 310.5 |
| 3 hrs. | 300.4 | 315.6 |
| 6 hrs. | 297.5 | 299.8 |
| * ND: under the measurement limit<br>Figures are averages over n = 3. | | |

Table 4-D

| Test No. | P I I |
|---|---|
| Example 10-D | 0.13 |
| Comparative Example 10-D | 1.35 |
| Example 11-D | 0.05 |
| Comparative Example 11-D | 0.95 |
| Example 12-D | 0.20 |
| Comparative Example 12-D | 1.15 |
| Example 13-D | 0.35 |
| Comparative Example 13-D | 1.95 |
| Example 14-D | 0.35 |
| Comparative Example 14-D | 1.85 |
| * Averages over n = 5. | |

Example 11-D

(Liniment drug)

| | |
|---|---|
| Morphine hydrochloride | 1 |
| Azacycloalkane derivative | 3 |
| L-menthol | 5 |
| Ethanol | 25 |
| Triethyl citrate | 27 |
| Isopropyl myrisate | 5 |
| Purified water | 34 |
| | 100 (wt.%) |

The above-mentioned composition was mixed as prescribed to give a liniment drug.

The results of skin irritation test are as shown in Table 4-D. As is clear from the comparison with the following Comparative Example 11-D, irritation is considerably reduced.

Comparative Example 11-D

(Liniment drug)

A comparative liniment drug was prepared in the same manner as above except that triethyl citrate in the Example 11-D was replaced with ethanol.

Example 12-D

(Gel-cream drug)

| | |
|---|---|
| Morphine hydrochloride | 5 |
| Azacycloalkane derivative | 3 |
| White vaseline | 5 |
| Cetanol | 4 |
| Liquid paraffin | 3 |
| Triethyl citrate | 15 |
| Paraoxymethyl benzoate | 0.2 |
| Carboxyvinyl polymer | 2 |
| Diisopropanol amine | 1 |
| Paraoxyethylene paraoxypropylene cetyl ether | 2 |
| Isopropanol | 20 |
| Purified water | 39.8 |
| | 100 (wt.%) |

So as to achieve the above composition, white vaseline, cetanol, liquid paraffin, triethyl citrate, azacycloalkane derivative, and paraoxyethylene paraoxypropylene cetyl ether were mixed, heated and dissolved, and then, paraoxymethyl benzoate, isopropanol and morphine hydrochloride dissolved and dispersed in previously heated purified water were added. The resultant mixture was then cooled to 50°C, and after addition and sufficient dispersion of carboxyvinyl polymer, added with diisopropanol amine to adjust pH to 4 to 8, thus giving a gel-cream drug.

The results of this skin irritation test are shown in Table 4-D. As is clear from the comparison wit the Comparative Example 12-D in Table 4-D, skin irritation is considerably reduced.

Comparative Example 12-D

(Gel-cream drug)

A comparative gel-cream drug was prepared in the same manner as above except that triethyl citrate in the Example 12-D was replaced with purified water.

Example 13-D

(Reservoir type patch drug)

The following components were combined:
1) Support: polyester film,
2) Medication storing layer: the composition used in the the Example 3,
3) Microporous membrane: duraguard
4) Adherent layer: acrylic adhesive (adherent layer surrounding the support),
and a peeloff paper was applied to the surfaces of the microporous membrane and the adherent layer to prepare a lamination.

The results of the skin irritation test are shown in Table 4-D, which reveal that skin irritation is largely reduced.

Comparative Example 13-D

(Reservoir type patch drug)

A comparative reservoir type patch drug was prepared in the same manner as above except that the contents of the medication storing layer in the Example 13-D was replaced with the composition of the Comparative Example 10-D. The results of the skin irritation test are shown in Table 4-D.

Example 14-D

(Patch drug)

First, 15 parts 1-hydroxycyclohexyl phenylketone were dissolved in 2,400 parts 2-hydroxyethyl methacrylate, and 35 parts tetraethylene glycol dimethacrylate were added and dissolved. Separately, 1,900 parts 5% salt water and 600 parts sulfopropyl methacrylate potassium salt were added and dissolved. Then, 2,000 parts glycerine, 2,000 parts isopropanol, 1,100 parts triethyl citrate, 110 parts morphine hydrochloride, and 300 parts azacycloalkane derivative were added to obtain a uniform solution. These solutions were mixed and bubbles were removed to obtain a uniform solution. The resultant solution was poured into a thickness of 300 um on a PET film kept horizontal, which had a thickness of 50 um and was provided with a frame to prevent the solution from flowing out. This was placed 15 cm below a 30 W chemical lamp while keeping it in a nitrogen atmosphere, and after irradiation of ultraviolet ray for five minutes, covered with a PET film, the hardened surface of which had been subjected to a stripping treatment, thus giving a patch drug.

The results of the skin irritation test are shown in Table 4-D, which reveal that skin irritation is largely reduced.

Comparative Example 14-D

(Patch drug)

A comparative patch drug was prepared in the same manner as above except that triethyl citrate in the Example 14-D was replaced with glycerine.

The results of the skin irritation test are shown in Table 4-D.

## INDUSTRIALLY USEFUL EFFECTS

The composition comprising trialkyl citrate, low-carbon alcohol and water of the present invention, an external drug formed by combining this composition with pharmaceutically active substances, and a composition and external drug combining this composition further with adsorption accelerators demonstrate the reducing action or the mitigating action of skin irritation having so far formed a drawback in conventional ones, and clearly have a remarkable effect against skin irritation so far feared. This has now permitted development of various drugs with the aim of percutaneous application excellent in safety. At the same time, an excellent absorption accelerating function is demonstrated in percutaneous absorption and it is possible to achieve drugs excellent also in pharmaceutical effects, so that it is possible to manufacture external drugs having the function of reducing and alleviating irritation and percutaneous absorption. The present invention thus provides pharmeceutically useful effects particularly in the form of percutaneous external drugs.

## Claims

1. A composition for mitigating skin irritation, which comprises trialkyl citrate, low-carbon alcohol and water.

2. A composition for mitigating skin irritation as claimed in Claim 1, wherein said composition contains 1 to 50 wt.% trialkyl citrate, 1 to 70 wt.% low-carbon alcohol, and 1 to 90 wt.% water.

3. A percutaneous external drug, which comprises trialkyl citrate, low-carbon alcohol, water and a pharmaceutically active substance.

4. A percutaneous external drug as claimed in Claim 3, wherein said drug contains 1 to 50 wt.% trialkyl citrate, 1 to 70 wt.% low-carbon alcohol, 1 to 90 wt.% water and 0.001 to 20 wt.% pharmaceutically active substance.

5. A composition for mitigating skin irritation, which contains a basal medium comprising trialkyl citrate, low-carbon alcohol and water, as well as an absorption accelerator.

6.  A composition for mitigating skin irritation as claimed in Claim 5, wherein said composition contains basal medium comprising 1 to 50 wt.% trialkyl citrate, 1 to 70 wt.% low-carbon alcohol, and 1 to 90 wt.% water, as well as 0.1 to 10 wt.% absorption accelerator.

7.  A composition for mitigating skin irritation, which contains a basal medium comprising trialkyl citrate, low-carbon alcohol and water, as well as an absorption accelerator and a pharmaceutically active substance.

8.  A percutaneous external drug, which contains a basal medium comprising 1 to 50 wt.% trialkyl citrate, 1 to 70 wt.% low-carbon alcohol and 1 to 90 wt.% water, added further with 0.1 to 10 wt.% absorption accelerator and 0.001 to 20 wt.% pharmaceutically active substance.

9.  A composition for mitigating skin irritation or a percutaneous external drug, wherein the absorption accelerator as claimed in any of Claims 5 to 8 is selected from the group consisting of terpene compounds, fatty acids, fatty acid alcohols, fatty acid esters, phenol derivatives, and azacyloalkane derivatives.

# INTERNATIONAL SEARCH REPORT

International Application No  PCT/JP92/00225

## I. CLASSIFICATION OF SUBJECT MATTER (if several classification symbols apply, indicate all) 6

According to International Patent Classification (IPC) or to both National Classification and IPC

Int. Cl$^5$  A61K47/14, A61K7/00

## II. FIELDS SEARCHED

### Minimum Documentation Searched 7

| Classification System | Classification Symbols |
|---|---|
| IPC | A61K47/06-47/14, A61K9/00-9/08, A61K9/70, A61K7/00-7/50 |

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 64-66107 (Kanebo, Ltd.), March 13, 1989 (13. 03. 89), (Family: none) | 1-7 |
| A | JP, A, 2-53718 (Beecham Group PLC), February 22, 1990 (22. 02. 90) & EP, A, 357186 | 1-7 |
| Y | JP, A, 1-113056 (Sekisui Chemical Co., Ltd.), May 1, 1989 (01. 05. 89), / (Family: none) | 8, 9 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| May 19, 1992 (19. 05. 92) | June 9, 1992 (09. 06. 92) |

| International Searching Authority | Signature of Authorized Officer |
|---|---|
| Japanese Patent Office | |

Form PCT/ISA/210 (second sheet) (January 1985)